# EUROPEAN PATENT APPLICATION

(11) **EP 4 119 179 A1**
(43) Date of publication of application: **18.01.2023**
(21) Application number: 22183761.0
(22) Date of filing: 08.07.2022
(51) Int. Cl.: A61M 16/08, F16L 11/00

(54) **MEDICAL TUBE**

(30) Priority: 16.07.2021 JP 2021117664
(71) Applicant: Magos Co., Ltd., Kawaguchi-shi, Saitama 332-0015 (JP)
(72) Inventor: NITTA, Kazufuku, Kawaguchi-shi, 332-0015 (JP); NGUYEN, Kim Quyen, Kawaguchi-shi, 332-0015 (JP)
(74) Representative: TBK

(57) **Abstract**

A medical tube is provided which can reduce the acoustic power level therein when air is supplied through the tube. The medical tube includes: a plurality of reference side bellows tube portions (2), each configured to be a flexible bellows-like tube including a plurality of reference rings (20) in series in an axial direction, the reference rings each extending annularly or helically to form a ring; and a plurality of deformed side tube portions (30), each configured to be a tube shaped differently from the reference side bellows tube portion. Here, the plurality of the reference side bellows tube portions and the plurality of the deformed side tube portions are alternately arranged in the axial direction. At least one of the reference side bellows tube portions has an axial length less than 112 mm.

## Description

### Technical Field

The present invention relates to a medical tube used in patient care.

### Background Art

Inspiratory tubes attached to respiratory masks are typically formed in a bellows-like shape (for example, see Patent Literature 1). The external shape of an inspiratory tube is corrugated so that outer crests (or annular protrusions) and inner troughs (or annular recesses) are alternately arranged. The outer crests and the inner troughs of the same shapes and sizes are formed repeatedly in the axial direction.

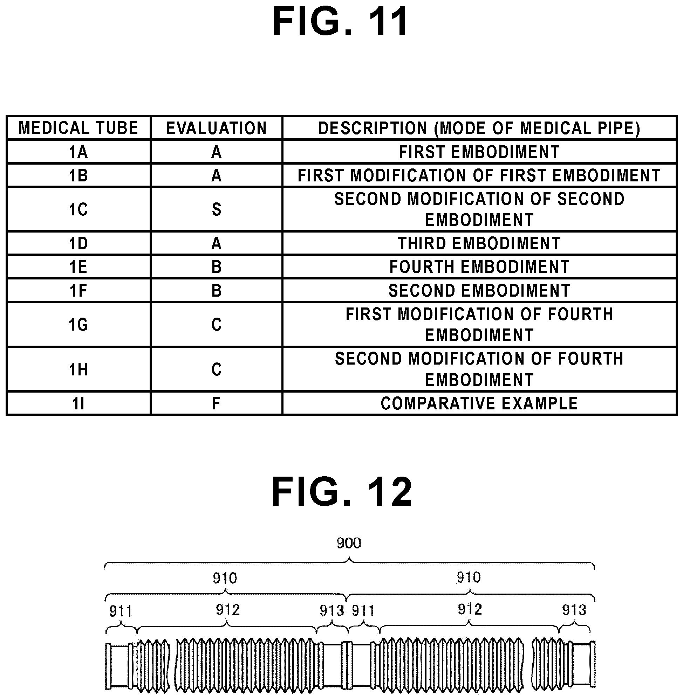

As shown in FIG. 12, an inspiratory tube 900 typically includes repetitions of unit shapes 910 of the same shape and size. A unit shape 910 includes a tube end portion 911, a bellows tube portion 912, and a tube end portion 913 connected in order. Adjoining tube end portions 911 and 913 are connected to repeat the unit shapes 910. The tube end portions 911 and 913 usually have an axial length of 19 mm each, and the bellows tube portion 912 has an axial length of 112 mm. The unit shape 910 thus has an axial length of 150 mm.

### Prior Art Literature

Patent Literature 1: Japanese Patent Application Laid-Open No. 2020-163168

### Summary of Invention

### Technical Problem

Supplying air through the inspiratory tube 900 having the above-described structure is noisy due to an increased acoustic power level inside the inspiratory tube 900.

In view of the above-described circumstances, it is an object of the present invention to provide a medical tube that can reduce the acoustic power level therein when air is supplied through the tube.

### Solution to Problem

A medical tube according to the present invention has been achieved to solve the above-described problem, and includes: a reference side bellows tube portion configured to be a flexible bellows-like tube including a plurality of reference rings in series in an axial direction, the reference rings each extending annularly or helically to form a ring; and a deformed side tube portion configured to be a tube shaped differently from the reference side bellows tube portion. Here, a plurality of the reference side bellows tube portions and a plurality of the deformed side tube portions are alternately arranged in the axial direction. At least one of the reference side bellows tube portions has an axial length less than 112 mm.

In the medical tube according to the present invention, the deformed side tube portions each include a plurality of deformed rings in series in the axial direction, the deformed rings each being shaped differently from the reference rings and extending annularly or helically to form a ring.

In the medical tube according to the present invention, the deformed rings have an inner wall-side maximum inner diameter smaller than that of the reference rings.

In the medical tube according to the present invention, the reference rings and the deformed rings have the same axial width.

In the medical tube according to the present invention, the deformed side tube portions are each configured to be a flexible bellows-like tube including the plurality of deformed rings in series in the axial direction.

In the medical tube according to the present invention, a deformed ridge shape has a ridge height lower than that of a reference ridge shape, with an inner wall-side protruding shape of a section of each reference ring taken along a center axis being defined as the reference ridge shape, and with an inner wall-side protruding shape of a section of each deformed ring taken along the center axis being defined as the deformed ridge shape.

In the medical tube according to the present invention, a deformed ridge shape has a large crest angle or curvature compared to that of a reference ridge shape, with an inner wall-side protruding shape of a section of each reference ring taken along a center axis being defined as the reference ridge shape, and with an inner wall-side protruding shape of a section of each deformed ring taken along the center axis being defined as the deformed ridge shape.

In the medical tube according to the present invention, the deformed side tube portions each include a cylindrical portion having an axial orthogonal cross section of perfectly circular shape perpendicular to a center axis of the medical tube on an inner wall side.

The medical tube according to the present invention includes a connection portion that, if cut halfway in the axial direction, takes a shape connectable to a medical member.

In the medical tube according to the present invention, the plurality of deformed side tube portions include ones having different axial lengths.

In the medical tube according to the present invention, the plurality of reference side bellows tube portions include ones having different axial lengths.

The medical tube according to the present invention includes at least three deformed side tube portions, and at least three deformed side tube portions arranged from one side to the other in the axial direction with the reference side bellows tube portions interposed therebetween increase or decrease in the axial length in order.

The medical tube according to the present invention includes at least three deformed side tube portions, and at least three deformed side tube portions arranged from one side to the other in the axial direction with the reference side bellows tube portions interposed therebetween increase or decrease in the number of deformed rings in order.

In the medical tube according to the present invention, the plurality of deformed side tube portions include a plurality of deformed side bellows tube portions each configured to be a flexible bellows-like tube including the plurality of deformed rings in series in the axial direction, and at least one connection portion, and the deformed side bellows tube portions and the connection portion are alternately arranged in the axial direction with the reference side bellows tube portions interposed therebetween.

A medical tube according to the present invention includes: a reference side bellows tube portion configured to be a flexible bellows-like tube including a plurality of reference rings in series in an axial direction, the reference rings each extending annularly or helically to form a ring; and a deformed side bellows tube portion configured to be a flexible bellows-like tube including a plurality of deformed rings in series in the axial direction, the deformed rings each extending annularly or helically to form a ring in a shape different from that of the reference rings. Here, the deformed rings have an inner wall-side maximum inner diameter smaller than that of the reference rings.

A medical tube according to the present invention has been achieved to solve the above-described problem, and includes: a reference side bellows tube portion configured to be a flexible bellows-like tube including a plurality of reference rings in series in an axial direction, the reference rings each extending annularly or helically to form a ring; and a deformed side tube portion configured to be a tube shaped differently from the reference side bellows tube portion. Here, a plurality of the reference side bellows tube portions and a plurality of the deformed side tube portions are alternately arranged in the axial direction. The plurality of reference side bellows tube portions include ones having different axial lengths.

A medical tube according to the present invention has been achieved to solve the above-described problem, and includes: a reference side bellows tube portion configured to be a flexible bellows-like tube including a plurality of reference rings in series in an axial direction, the reference rings each extending annularly or helically to form a ring; and a deformed side tube portion configured to be a tube shaped differently from the reference side bellows tube portion. Here, a plurality of the reference side bellows tube portions and a plurality of the deformed side tube portions are alternately arranged in the axial direction. The plurality of deformed side tube portions include ones having different axial lengths.

A medical tube according to the present invention includes: a plurality of reference side bellows tube portions each configured to be a flexible bellows-like tube including a plurality of reference rings in series in an axial direction, the reference rings each extending annularly or helically to form a ring; a plurality of deformed side bellows tube portions each configured to be a tube shaped differently from the reference side bellows tube portions, the tube being a flexible bellows-like tube including a plurality of deformed rings in series in the axial direction, the deformed rings each extending annularly or helically to form a ring in a different shape from that of the reference rings; and a plurality of tube end portions each configured to be a tube shaped differently from the reference side bellows tube portions and including a cylindrical portion having an axial orthogonal cross section of perfectly circular shape perpendicular to a center axis of the medical tube. Here, at least one of the reference side bellows tube portions has an axial length greater than those of the deformed side bellows tube portions. The reference side bellows tube portions and the deformed side bellows tube portions are alternately arranged in the axial direction. A medical tube piece adjoins the tube end portions at respective ends, where a configuration in which the plurality of reference side bellows tube portions and at least one of the deformed side bellows tube portions are alternately arranged in the axial direction being defined as the medical tube piece. A base of a reference ridge shape and a base of a deformed ridge shape are located on a common imaginary reference cylindrical surface of cylindrical shape about a center axis, with an inner wall-side protruding shape of a cross section of each reference ring taken along the center axis being defined as the reference ridge shape, and with an inner wall-side protruding shape of a cross section of each deformed ring taken along the center axis being defined as the deformed ridge shape. The deformed ridge shape has a ridge height lower than that of the reference ridge shape.

In the medical tube according to the present invention, inner wall-side innermost parts of the tube end portions have an inner diameter the same as a diameter of the imaginary reference cylindrical surface. In the medical tube according to the present invention, at least one of the tube end portions between adjoining ones of the medical tube pieces includes a connection portion that, if cut, takes a shape connectable to a medical member.

A medical tube according to the present invention includes: a plurality of reference side bellows tube portions each configured to be a flexible bellows-like tube including a plurality of reference rings in series in an axial direction, the reference rings each extending annularly or helically to form a ring; and at least one of deformed side bellows tube portions each configured to be a tube shaped differently from the reference side bellows tube portions, the tube being a flexible bellows-like tube including a plurality of deformed rings in series in the axial direction, the deformed rings each extending annularly or helically to form a ring in a shape different from the reference rings. Here, the reference side bellows tube portions and the deformed side bellows tube portions are alternately arranged in the axial direction. A sum of an axial length of the reference side bellows tube portions included in the medical tube is greater than that of the deformed side bellows tube portions included in the medical tube. A base of a reference ridge shape and a base of a deformed ridge shape are located on a common imaginary reference cylindrical surface of cylindrical shape about a center axis, with an inner wall-side protruding shape of a cross section of each reference ring taken along the center axis being defined as the reference ridge shape, and with an inner wall-side protruding shape of a cross section of each deformed ring taken along the center axis being defined as the deformed ridge shape. The deformed ridge shape has a ridge height lower than that of the reference ridge shape.

The medical tube according to the present invention includes a plurality of tube end portions each configured to be a tube shaped differently from the reference side bellows tube portions and including a cylindrical portion having an axial orthogonal cross section of perfectly circular shape perpendicular to a center axis of the medical tube. A medical tube piece adjoins the tube end portions at respective ends, where a configuration in which the plurality of reference side bellows tube portions and at least one of the deformed side bellows tube portions are alternately arranged in the axial direction being defined as the medical tube piece. A sum of a axial length of the plurality of reference side bellows tube portions included in the medical tube piece is greater than that of the deformed side bellows tube portion included in the medical tube piece.

### Advantageous Effects of Invention

The medical tube according to the present invention can provide an excellent effect that the acoustic power level inside the tube can be reduced when air is supplied into the tube.

### Brief Description of Drawings

FIG. 1(A) is a diagram showing a medical tube according to a first embodiment of the present invention. FIG. 1(B) is a cross-sectional view of the medical tube according to the first embodiment of the present invention, taken along an axial direction of the medical tube.
FIG. 2(A) is a cross-sectional view of reference rings and deformed rings of the medical tube according to the first embodiment of the present invention, taken along the axial direction of the medical tube. FIG. 2(B) is a cross-sectional view of a tube end portion of the medical tube according to the first embodiment of the present invention, taken along the axial direction of the medical tube. FIG. 2(C) is an axial orthogonal cross-sectional view of the tube end portion of the medical tube according to the first embodiment of the present invention.
FIGS. 3(A) and 3(B) are cross-sectional views of modifications of the reference rings and deformed rings of the medical tube according to the first embodiment of the present invention, taken along the axial direction of the medical tube.
FIG. 4(A) is a diagram showing a medical tube configured by connecting a plurality of medical tube pieces according to the first embodiment of the present invention. FIG. 4(B) is a diagram showing a medical tube configured by connecting a plurality of conventional medical tube pieces.

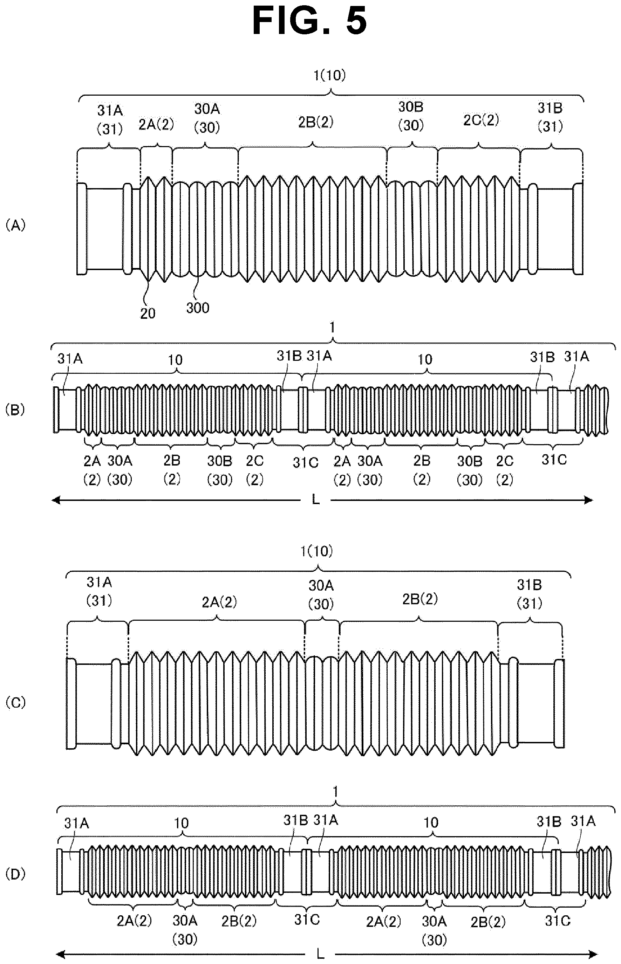

FIG. 5(A) is a diagram showing a medical tube (medical tube piece) according to a first modification of the first embodiment of the present invention. FIG. 5(B) is a diagram showing a medical tube configured by connecting a plurality of medical tube pieces according to the first modification of the first embodiment of the present invention. FIG. 5(C) is a diagram showing a medical tube(medical tube piece) according to a second modification of the first embodiment of the present invention. FIG. 5(D) is a diagram showing a medical tube configured by connecting a plurality of medical tube pieces according to the second modification of the first embodiment of the present invention.
FIG. 6(A) is a diagram showing a medical tube according to a second embodiment of the present invention. FIG. 6(B) is a cross-sectional view of reference rings and deformed rings of the medical tube according to the second embodiment of the present invention, taken along the axial direction of the medical tube. FIGS. 6(C) and 6(D) are diagrams showing modifications of the medical tube according to the second embodiment of the present invention.
FIG. 7(A) is a diagram showing a medical tube according to a third embodiment of the present invention. FIG. 7(B) is a diagram showing a modification of the medical tube according to the third embodiment of the present invention.
FIG. 8(A) is a diagram showing a medical tube according to a fourth embodiment of the present invention. FIG. 8(B) is a diagram showing a modification of the medical tube according to the fourth embodiment of the present invention.

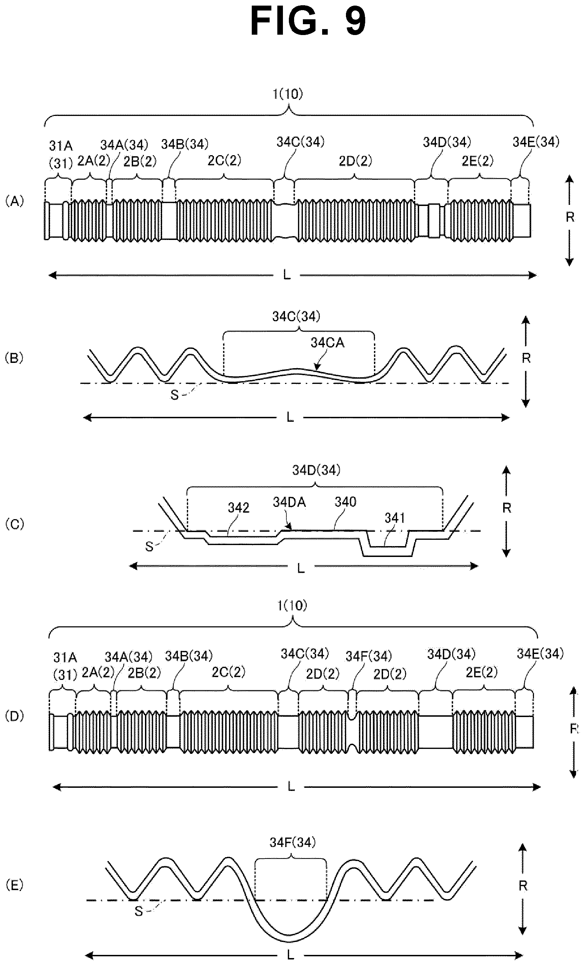

FIG. 9(A) is a diagram showing a first modification of the medical tube according to the fourth embodiment of the present invention. FIGS. 9(B) and 9(C) are cross-sectional views of cylindrical portions of the first modification of the medical tube according to the fourth embodiment of the present invention, taken along the axial direction of the medical tube. FIG. 9(D) is a diagram showing a second modification of the medical tube according to the fourth embodiment of the present invention. FIG. 9(E) is a cross-sectional view of a cylindrical portion of the second modification of the medical tube according to the fourth embodiment of the present invention, taken along the axial direction of the medical tube.
FIGS. 10(A) to 10(I) are diagrams showing the results of a simulation of the acoustic power levels of ten types of medical tubes, conducted using predetermined simulation software.
FIG. 11 is a table showing evaluations of the simulation results shown in FIG. 10.
FIG. 12 is a diagram showing a conventional gas supply tube.

### Description of Embodiments

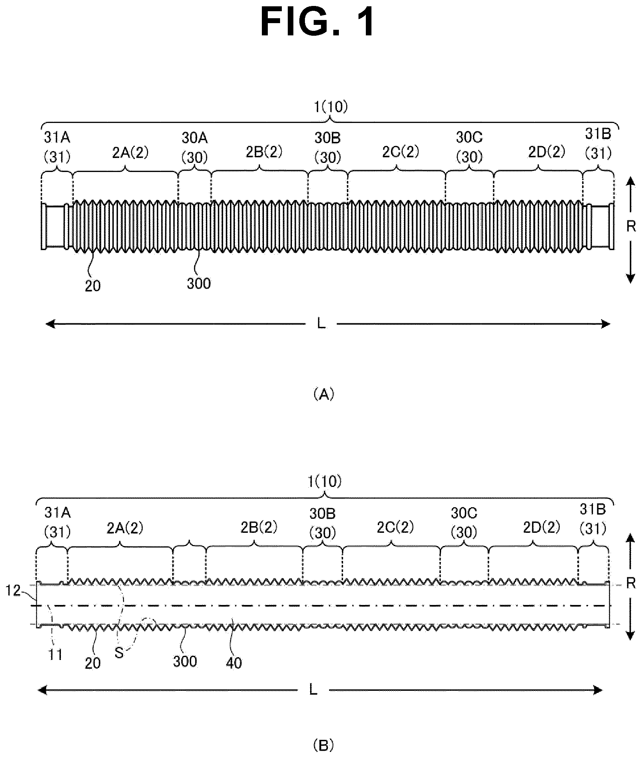

Embodiments of the present invention will be described below with reference to the accompanying drawings. The present invention includes a medical tube, a medical tubing and air delivery conduit. FIGS. 1(A) to 11 show examples of embodiment of the invention. In the diagrams, portions designated by the same reference numerals as in FIGS. 1(A) and 1(B) represent the same components.

### <First Embodiment>

A medical tube 1 according to a first embodiment of the present invention will be described with reference to FIG. 1. The medical tube 1 according to the present embodiment is used as an inspiratory tube or an air delivery conduit of a respiratory assistance device such as an artificial respirator, for example. The medical tube 1 according to the present embodiment includes a plurality of reference side bellows tube portions 2 and a plurality of deformed side tube portions 3.

### <Reference Side Bellows Pipe Portions>

As shown in FIG. 1(A), each reference side bellows tube portion 2 is configured to be a flexible bellows-like tube including a plurality of reference rings 20(may be called the reference annular ring 20) in series in an axial direction L. The reference rings 20 each extend annularly or helically to form a ring. In the diagrams of the present application, the axial direction of the medical tube 1 is referred to as an axial direction L. A radial direction of the medical tube 1 orthogonal to the axial direction L in a cross-sectional view where the medical tube 1 is sectioned along the axial direction L is referred to as a radial direction R.

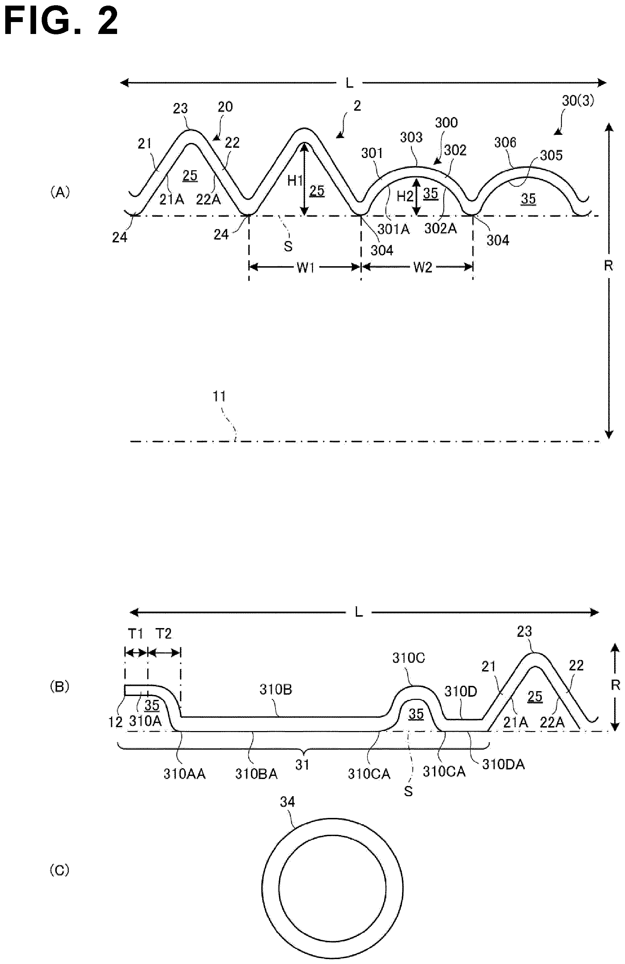

The reference rings 20 serve as a refence among a plurality of rings constituting the medical tube 1. The reference rings 20 extend circumferentially all around the medical tube 1. As shown in FIGS. 1(B) and 2(A), each reference ring 20 has a protruding shape or ridge shape (hereinafter, referred to as a reference ridge shape) with the inner wall side (inner peripheral side) or outer wall side (outer peripheral side) of the section taken along the axial direction L convex outward in the radial direction R. The reference rings 20 constituting the reference side bellows tube portions 2 have the reference ridge shapes of the same size and shape.

Specifically, as shown in FIG. 2(A), each reference ring 20 includes annular wall portions 21 and 22 that are connected at a crest 23 of the reference ridge shape, with their respective inner wall surfaces 21A and 22A opposed to each other in the axial direction L. The annular wall portions 21 and 22 are formed in an annular shape that extends along the circumferential direction of the medical tube 1. The annular wall portions 21 and 22 are inclined to be closer to each other in the axial direction L toward the outer region of the radial direction R. As shown in FIG. 2(A), the annular wall portions 21 and 22 extend straight with a substantial constant thickness from bases 24 to the crest 23 of the reference ridge shape. As a result, the crest 23 of the reference ring 20 is configured to be pointed. The bases 24 of the reference ridge shape refer to the innermost parts of the annular wall portions 21 and 22 closest to a center axis 11 of the medical tube 1 in the radial direction R. The crest 23 of the reference ridge shape refers to the outermost parts of the annular wall portions 21 and 22 farthest from the center axis 11 of the medical tube 1 in the radial direction R.

### <Deformed Side Pipe Portions>

The deformed side tube portions 3 are configured to be tubes shaped differently from the reference side bellows tube portions 2. In the present embodiment, the deformed side tube portions 3 include deformed side bellows tube portions 30 and tube end portions 31.

### <Deformed Side Bellows Pipe Portions>

The deformed side bellows tube portions 30 are each configured to be a flexible bellows-like tube including a plurality of deformed rings 300(may be called the deformed annular ring 300) in series in the axial direction L. Each deformed ring 300 has a different size and/or shape from that of the reference ring 20 and extends annularly or helically to form a ring. As shown in FIGS. 1(B) and 2(A), the section of the deformed ring 300 taken along the axial direction L has a ridge shape (hereinafter, referred to as a deformed ridge shape) convex outward in the radial direction R. The deformed ring 300 extends circumferentially all around the medical tube 1. The deformed rings 300 constituting the deformed side bellows tube portions 30 have the deformed ridge shapes of the same size and shape.

Specifically, as shown in FIG. 2(A), each deformed ring 300 includes a pair of annular wall portions 301 and 302 that are connected at a crest 303 of the deformed ridge shape, with their inner wall surfaces 301A and 302A opposed to each other in the axial direction L. The annular wall portions 301 and 302 are formed in an annular shape that extends circumferentially all around the medical tube 1. The annular wall portions 301 and 302 are inclined to be closer to each other in the axial direction L toward the outer region of the radial direction R.

As shown in FIG. 2(A), the annular wall portions 301 and 302 each extend while curving to be radially convex outward, with a substantially constant thickness from a base 304 to the crest 303 of the deformed ridge shape. As a result, the crest 303 of the deformed ring 300 is configured to be curved and round. Each of the annular wall portions 301 and 302 of the deformed ring 300 desirably has the same thickness as that of the annular wall portions 21 and 22 of the reference ring 20.

In the present embodiment, as shown in FIG. 2(A), the bases 304 of the deformed ridge shapes and the bases 24 of the reference ridge shapes are located at equal distances in the radial direction R from the center axis 11 of the medical tube 1. In other words, the bases 304 of the deformed ridge shapes and the bases 24 of the reference ridge shapes are located on an imaginary reference cylindrical surface S of cylindrical shape about the center axis 11 of the medical tube 1. This imaginary reference cylindrical surface S ensures a stable flow rate. Note that at least either the bases 304 of the deformed ridge shapes or the bases 24 of the reference ridge shapes may be located in a position displaced from the imaginary reference cylindrical surface S in the radial direction R. The medical tube 1 as described above is also encompassed by the scope of the present invention.

As shown in FIG. 2(A), the deformed ridge shapes of the deformed rings 300 in the present embodiment have an inner wall-side ridge height H2 smaller than an inner wall-side ridge height H1 of the reference ridge shapes of the reference rings 20. The inner wall-side ridge heights of the reference ridge shapes and the deformed ridge shapes refer to the heights of the reference ridge shapes and the deformed ridge shapes in the radial direction R with respect to their respective bases 24 and 304, or the imaginary reference cylindrical surface S. The sum of twice the ridge height H2 of the deformed rings 300 and the inner diameter of the imaginary reference cylindrical surface S may be referred to as the inner wall-side maximum inner diameter of the deformed rings 300. Similarly, the sum of twice the ridge height H1 of the reference rings 20 and the inner diameter of the imaginary reference cylindrical surface S may be referred to as the inner wall-side maximum inner diameter of the reference rings 20.

In the present embodiment, as shown in FIG. 2(A), the deformed ridge shapes of the deformed rings 300 have an inner wall-side ridge width W2 substantially the same as an inner wall-side ridge width W1 of the reference ridge shapes of the reference rings 20. The ridge widths W1 and W2 of the reference ridge shapes and the deformed ridge shapes refer to the widths (axial widths) of the reference ridge shapes and the deformed ridge shapes in the axial direction L. Note that the ridge width W2 of the deformed ridge shapes of the deformed rings 300 may be greater or smaller than the ridge width W1 of the reference ridge shapes of the reference rings 20. The medical tube 1 as described above is also encompassed by the scope of the present invention. In other words, the concept that the reference rings 20 and the deformed rings 300 are different in shape desirably includes at least any one of the following: that the ridge widths W1 and W2 are different, that the ridge heights H1 and H2 are different, that the crests have different diameters, that the troughs have different diameters, and that the ridges themselves are different in shape (have different shapes such as a straight ridge, a curved ridge, and a trapezoidal ridge, have a difference between the curvatures of the curved ridges, and so on) .

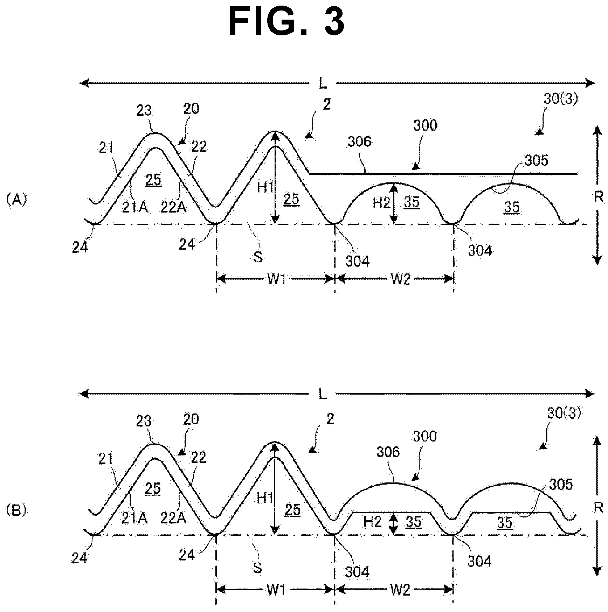

In the above-described description, an inner peripheral surface 305 and an outer peripheral surface 306 of each deformed ring 300 are configured to be curved surfaces radially convex outward so that the entire deformed ring 300 has a substantially constant thickness. Both the inner and outer peripheral surfaces 305 and 306 are thereby formed in a ridge shape. However, this is not restrictive. A higher priority is given to the ridge shape of the inner peripheral surface 305. For example, as shown in FIG. 3(A), the deformed ring 300 may include an outer peripheral surface 306 that is a cylindrical surface analogous to and greater in diameter than the imaginary reference cylindrical surface S, and an inner peripheral surface 305 that is a curved surfaces(recessed surface) radially convex outward. In such a case, the deformed ring 300 is thick at the troughs of the ridge shape. In another example, as shown in FIG. 3(B), the deformed ring 300 may be configured so that the inner peripheral surface 305 is a recessed surface radially convex outward in a trapezoidal shape in section. Such configurations are also encompassed by the scope of the present invention.

### <Pipe End Portions>

The tube end portions 31 have a shape connectable to a medical member. As shown in FIGS. 1(A) and 1(B), the tube end portions 31 are located at the ends of the medical tube 1. Examples of the medical member include a mask that covers a patient's mouth and nose. As shown in FIG. 2(B), each tube end portion 31 is a tube including deformed rings 310A to 310D arranged in order in the axial direction L. The deformed rings 310A to 310D each have a different size and/or shape from that of the reference rings 20 and extend annually to form a ring.

As shown in FIG. 2(B), the deformed ring 310A is located at an end of the medical tube 1, and includes a first interval T1 and a second interval T2. The first interval T1 extends from an end of the deformed ring 310A farther from the deformed ring 310B (far-side end) toward the deformed ring 310B in parallel with the axial direction L. The second interval T2 decreases in diameter (contracts) while extending in the axial direction L from the end (terminal end) of the first interval T1 opposite the far-side end toward the deformed ring 310B. The second interval T2 of the deformed ring 310A is curved to be convex outward. The end (terminal end) of the second interval T2 of the deformed ring 310A includes a base 310AA of the deformed ring 310A. The base 310AA of the deformed ring 310A is the inner wall-side innermost part of the deformed ring 310A closest to the center axis 11 of the medical tube 1 in the radial direction R. The base 310AA is located at the boundary between the deformed ring 310A (second interval T2) and the deformed ring 310B. The base 310AA of the deformed ring 310A is located on the imaginary reference cylindrical surface S. In other words, the inner diameter of the inner wall-side innermost part of the deformed ring 310A (minimum inner diameter of the deformed ring 310A) is the same as the diameter of the imaginary reference cylindrical surface S. In the present embodiment, as shown in FIG. 2(B), the deformed ring 310A is substantially L-shaped so that its section taken along the axial direction L is convex outward in the radial direction R. The end rim of the deformed ring 310A on the inner peripheral side forms an opening 12 of the medical tube 1. In the present embodiment, the inner wall-side height of the deformed ring 310A in the radial direction R is substantially the same as the inner wall-side ridge height of the deformed ridge shape of the deformed ring 310C. However, this is not restrictive, and the inner wall-side height may be smaller or greater.

As shown in FIG. 2(C), the deformed rings 310B and 310D include a cylinder portion 34 having an axial orthogonal cross section of perfectly circular shape perpendicular to the center axis 11 of the medical tube 1. The axial length of the deformed ring 310B in the axial direction L is greater than that of the deformed ring 310D. As shown in FIG. 2(B), an inner wall surface 310BA of the deformed ring 310B and an inner wall surface 310DA of the deformed ring 310D are located on the imaginary reference cylindrical surface S. In other words, the inner diameters of the inner wall-side innermost parts of the deformed rings 310B and 310D closest to the center axis 11 of the medical tube 1 in the radial direction R (the minimum inner diameters of the deformed rings 310B and 310D) are the same as the diameter of the imaginary reference cylindrical surface S.

The deformed ring 310C has a deformed ridge shape having a different size and shape from those of the deformed rings 300. In the present embodiment, the deformed ridge shape of the deformed ring 310C is smaller in the lateral width and lower in the ridge height than the deformed ridge shape of the deformed rings 300. However, this is not restrictive. In other words, the deformed ridge shape of the deformed ring 310C may have the same size and shape as those of the deformed ridge shape of the deformed rings 300, or greater in the lateral width or higher in the ridge height than the deformed ridge shape of the deformed rings 300. In any case, the inner wall-side maximum inner diameters of the deformed side tube portions 3 (deformed side bellows tube portions 30 and tube end portions 31) are desirably smaller than that of the reference rings 20. Two bases 310CA that are the innermost parts of the deformed ring 310C closest to the center axis 11 of the medical tube 1 in the radial direction R are located at respective ends of the deformed ring 310C in the axial direction L. The bases 310CA are located at the boundary between the deformed ring 310C and the deformed ring 310B and at the boundary between the deformed ring 310C and the deformed ring 310D. As shown in FIG. 2(B), the two bases 310CA of the deformed ring 310C are located on the imaginary reference cylindrical surface S. In other words, the inner diameter of the inner wall-side innermost parts of the deformed ring 310C closest to the center axis 11 of the medical tube 1 in the radial direction R (minimum inner diameter of the deformed ring 310C) is the same as the diameter of the imaginary reference cylindrical surface S.

As described above, the innermost parts of the tube end portions 31 closest to the center axis 11 of the medical tube 1 in the radial direction R (the base 310AA of the deformed ring 310A, the inner wall surface 310BA of the deformed ring 310B, the two bases 310CA of the deformed ring 310C, and the inner wall surface 310DA of the deformed ring 310D) are located on the imaginary reference cylindrical surface S. In other words, the inner diameters of the inner wall-side innermost parts of the tube end portions 31 closest to the center axis 11 of the medical tube 1 in the radial direction R (the minimum inner diameter of the tube end portions 31) are the same as the diameter of the imaginary reference cylindrical surface S.

The tube end portions 31 are not limited to the above-described shape, and may have other shapes connectable to a medical member.

### <Overall Configuration of Medical Pipe>

As shown in FIGS. 1(A) and 1(B), the medical tube 1 includes the plurality of reference side bellows tube portions 2 and the plurality of deformed side tube portions 3 alternately arranged in the axial direction L. As a result, each deformed side bellows tube portion 30 is located between two reference side bellows tube portions 2. The plurality of reference side bellows tube portions 2 include ones having the same length in the axial direction L (hereinafter, referred to as an axial length) and ones having different axial lengths. The plurality of deformed side tube portions 3 have respective different axial lengths. In order to distinguish them from one another, the reference side bellows tube portions 2 will be referred to, in order from the left in FIG. 1(A), as a first reference side bellows tube portion 2A, a second reference side bellows tube portion 2B, a third reference side bellows tube portion 2C, and a fourth reference side bellows tube portion 2D. The deformed side bellows tube portions 30 will be referred to, in order from the left in FIG. 1(A), as a first deformed side bellows tube portion 30A, a second deformed side bellows tube portion 30B, and a third deformed side bellows tube portion 30C. As shown in FIGS. 1(A) and 1(B), the medical tube 1 includes a tube end portion 31A, the first reference side bellows tube portion 2A, the first deformed side bellows tube portion 30A, the second reference side bellows tube portion 2B, the second deformed side bellows tube portion 30B, the third reference side bellows tube portion 2C, the third deformed side bellows tube portion 30C, the fourth reference side bellows tube portion 2D, and a tube end portion 31B connected in order.

In the plurality of reference side bellows tube portions 2, the second reference side bellows tube portion 2B and the third reference side bellows tube portion 2C have the same axial length. The other reference side bellows tube portions 2 have respective different axial lengths.

The first reference side bellows tube portion 2A, the second reference side bellows tube portion 2B (third reference side bellows tube portion 2C), and the fourth reference side bellows tube portion 2D decrease in the number of connected reference rings 20 in this order. Specifically, the first reference side bellows tube portion 2A includes a series of 13 reference rings 20. The second reference side bellows tube portion 2B (third reference side bellows tube portion 2C) includes a series of 12 reference rings 20. The fourth reference side bellows tube portion 2D includes a series of 11 reference rings 20. As a result, the first reference side bellows tube portion 2A, the second reference side bellows tube portion 2B (third reference side bellows tube portion 2C), and the fourth reference side bellows tube portion 2D decrease in axial length in this order. In other words, the medical tube 1 according to the present embodiment decreases in the number of connected reference rings 20 and in axial length from the first reference side bellows tube portion 2A to the fourth reference side bellows tube portion 2D.

The first reference side bellows tube portion 2A, the second reference side bellows tube portion 2B (third reference side bellows tube portion 2C), and the fourth reference side bellows tube portion 2D are not limited to the above-described configuration, and may all have different axial lengths.

The first deformed side bellows tube portion 30A, the second deformed side bellows tube portion 30B, and the third deformed side bellows tube portion 30C increase in the number of connected deformed rings 300 in this order. In FIG. 1(A), the first deformed side bellows tube portion 30A includes a series of four deformed rings 300. The second deformed side bellows tube portion 30B includes a series of five deformed rings 300. The third deformed side bellows tube portion 30C includes a series of six deformed rings 300. As a result, the first deformed side bellows tube portion 30A, the second deformed side bellows tube portion 30B, and the third deformed side bellows tube portion 30C increase in axial length in this order. In other words, the medical tube 1 according to the present embodiment increases in the number of connected deformed rings 300 and in axial length from the first deformed side bellows tube portion 30A to the third deformed side bellows tube portion 30C.

The first deformed side bellows tube portion 30A, the second deformed side bellows tube portion 30B, and the third deformed side bellows tube portion 30C are not limited to the above-described configuration, and ones having the same axial length may be included.

The reference side bellows tube portions 2 (first reference side bellows tube portion 2A, second reference side bellows tube portion 2B, third reference side bellows tube portion 2C, and fourth reference side bellows tube portion 2D) are greater in axial length than the deformed side bellows tube portions 30 (first deformed side bellows tube portion 30A, second deformed side bellows tube portion 30B, and third deformed side bellows tube portion 30C). In other words, in the medical tube 1 (medical tube piece 10 be described below) according to the present embodiment, the total of the axial lengths of the first reference side bellows tube portion 2A, the second reference side bellows tube portion 2B, the third reference side bellows tube portion 2C, and the fourth reference side bellows tube portion 2D is greater than that of the axial lengths of the first deformed side bellows tube portion 30A, the second deformed side bellows tube portion 30B, and the third deformed side bellows tube portion 30C. Moreover, the medical tube 1 (medical tube piece 10 to be described below) according to the present embodiment includes more reference rings 20 than deformed rings 300. In the present embodiment, the second deformed side bellows tube portion 30B can be regarded as being located substantially in the center of the medical tube 1. The first deformed side bellows tube portion 30A and the third deformed side bellows tube portion 30C can be regarded as being located at positions displaced from the substantial center toward the ends of the medical tube 1 and where the medical tube 1 is divided into substantially equal quarters. That is, in the medical tube 1 according to the present embodiment, the reference side bellows tube portions 2 and the deformed side bellows tube portions 30 are arranged with their center positions slightly displaced from the equally dividing positions of the medical tube 1.

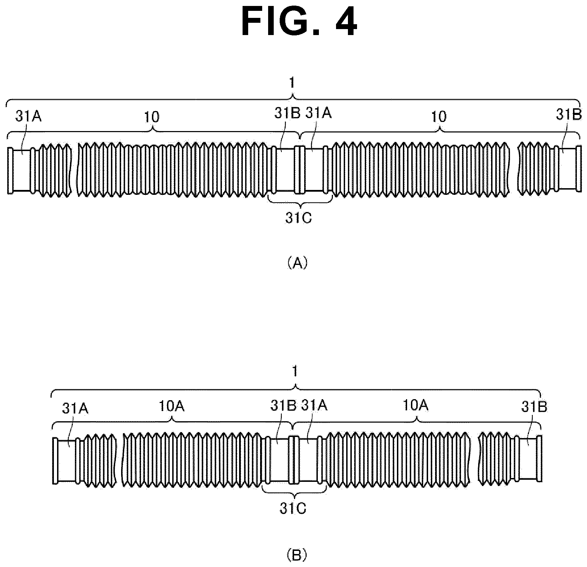

Assuming the above-described configuration as one medical tube piece 10, a medical tube 1 formed by connecting medical tube pieces 10 in series as shown in FIG. 4(A) is also encompassed by the scope of the present invention. If medical tube pieces 10 are connected in series, a connection portion 31C where tube end portions 31B and 31A are connected to each other is formed between adjoining medical tube pieces 10.

In adjusting the length of a medical tube 1 including connection portions 31C, a connection portion 31C is cut in the middle. Cutting off the connection portion 31C in the middle produces the tube end portions 31B and 31A.

As shown in FIG. 2(B), the innermost parts of the medical tube 1 closest to the center axis 11 of the medical tube 1 in the radial direction R (the bases 24 of the reference ridge shapes, the bases 304 of the deformed ridge shapes, and the innermost parts of the tube end portions 31 (corresponding to the bases of the tube end portions 31)) are located on the imaginary reference cylindrical surface S. In other words, the inner diameter of the inner wall-side innermost parts of the medical tube 1 (the minimum inner diameter of the medical tube 1) is the same as the diameter of the imaginary reference cylindrical surface S.

The tube end portions 31A and 31B have an axial length less than that of the medical tube piece 10, desirably less than or equal to 1/10 that of the medical tube piece 10, more desirably less than or equal to 1/15 that of the medical tube piece 10. The axial length of the tube end portions 31A and 31B may be less than that of each of the reference side bellows tube portions 2. The axial length of the tube end portions 31A and 31B may be less than that of each of the deformed side bellows tube portions 30.

### <Axial Lengths of Medical Pipe>

In the present embodiment shown in FIG. 1, the axial length of the tube end portions 31A is desirably less than or equal to 30 mm, and is 19 mm here. The axial length of the first reference side bellows tube portion 2A is desirably less than 112 mm, and is 64.7 mm here. The axial length of the first deformed side bellows tube portion 30A is desirably less than or equal to 40 mm, and is 20 mm here. The axial length of the second reference side bellows tube portion 2B is desirably less than 112 mm, and is 60 mm here. The axial length of the second deformed side bellows tube portion 30B is desirably less than or equal to 40 mm, and is 25 mm here. The axial length of the third reference side bellows tube portion 2C is desirably less than 112 mm, and is 60 mm here. The axial length of the third deformed side bellows tube portion 30C is desirably less than or equal to 40 mm, and is 30 mm here. The axial length of the fourth reference side bellows tube portion 2D is desirably less than 112 mm, and is 55 mm here. The axial length of the tube end portion 31B is desirably less than or equal to 30 mm, and is 19 mm here.

The axial length of the reference rings 20 and that of the deformed rings 300 are desirably less than or equal to 10 mm, and are 5 mm here. Note that the reference ring 20 continuous with the tube end portion 31A has an axial length of 4.7 mm. The inner wall-side ridge height of the reference ridge shape of the reference rings 20 is desirably less than or equal to 5 mm, and is 3.5 mm here. The inner wall-side ridge height of the deformed ridge shape of the deformed rings 300 is desirably less than or equal to 4.5 mm, and is 2.7 mm here. A difference between the ridge height of the reference ridge shape and that of the deformed ridge shape is desirably greater than or equal to 0.5 mm. The diameter of the imaginary reference cylindrical surface S (the inner diameter of the medical tube 1) is 22 mm.

As shown in FIG. 4(B), a conventionally used medical tube 1 includes a series of medical tube pieces 10A each including a tube end portion 31A, a reference side bellows tube portion 2, and a tube end portion 31B connected in order. Such medical tube pieces 10A typically have an axial length of 150 mm. Both tube end portions 31A and 31B included in a medical tube piece 10A each have an axial length of 19 mm, and the reference side bellows tube portion 2 has an axial length of 112 mm.

In the present embodiment, the axial lengths of the reference side bellows tube portions 2 (first reference side bellows tube portion 2A, second reference side bellows tube portion 2B, third reference side bellows tube portion 2C, and fourth reference side bellows tube portion 2D) are less than 112 mm. However, the medical tube 1 may include a reference side bellows tube portion 2 having an axial length of 112 mm or more.

### <Modifications of First Embodiment>

Modifications of the medical tube 1 according to the present embodiment will be described with reference to FIGS. 5(A) to 5(D). FIG. 5(A) shows a medical tube 1 according to a first modification. This medical tube 1 includes a tube end portion 31A, a first reference side bellows tube portion 2A, a first deformed side bellows tube portion 30A, a second reference side bellows tube portion 2B, a second deformed side bellows tube portion 30B, a third reference side bellows tube portion 2C, and a tube end portion 31B connected in order. In the medical tube 1 according to the first modification, both the number of reference side bellows tube portions 2 and the number of deformed side bellows tube portions 3 are one less than in the medical tube 1 according to the present embodiment.

Again, in the first modification, the first reference side bellows tube portion 2A, the second reference side bellows tube portion 2B, and the third reference side bellows tube portion 2C have respective different axial lengths. In other words, the first reference side bellows tube portion 2A, the second reference side bellows tube portion 2B, and the third reference side bellows tube portion 2C include series of respective different numbers of reference rings 20.

In the medical tube 1 according to the first modification, the first reference side bellows tube portion 2A includes a series of two reference rings 20. The second reference side bellows tube portion 2B includes a series of nine reference rings 20. The third reference side bellows tube portion 2C includes a series of five reference rings 20. That is, in the medical tube 1 according to the first modification, the number of reference rings 20 included in a reference side bellows tube portion 2 varies along the axial direction. As a result, in the medical tube 1 according to the first modification, the axial lengths of the reference side bellows tube portions 2 vary along the axial direction.

Again, in the first modification, the first deformed side bellows tube portion 30A and the second deformed side bellows tube portion 30B have respective different axial lengths. In other words, the first deformed side bellows tube portion 30A and the second deformed side bellows tube portion 30B include series of respective different numbers of deformed rings 300. In the medical tube 1 according to the first modification, the first deformed side bellows tube portion 30A includes a series of four deformed rings 300, and the second deformed side bellows tube portion 30B includes a series of three deformed rings 300. In the first modification, the first deformed side bellows tube portion 30A and the second deformed side bellows tube portion 30B can be regarded as being located on both sides of the center of the medical tube 1.

The axial length of the first reference side bellows tube portion 2A is less than those of the first deformed side bellows tube portion 30A and the second deformed side bellows tube portion 30B. The axial lengths of the first deformed side bellows tube portion 30A and the second deformed side bellows tube portion 30B are less than those of both the second reference side bellows tube portion 2B and the third reference side bellows tube portion 2C. In other words, the medical tube 1 according to the present invention includes at least one reference side bellows tube portion 2 (e.g., second reference side bellows tube portion 2B or third reference side bellows tube portion 2C) having an axial length greater that those of the deformed side bellows tube portions 30. Such a medical tube 1 may include a reference side bellows tube portion 2 having an axial length less than those of the deformed side bellows tube portions 30 like the above-described first reference side bellows tube portion 2A.

The total of the axial lengths of the first reference side bellows tube portion 2A, the second reference side bellows tube portion 2B, and the third reference side bellows tube portion 2C is greater than that of the axial lengths of the first deformed side bellows tube portion 30A and the second deformed side bellows tube portion 30B. In other words, A sum of an axial length of the reference side bellows tube portions included in the medical tube 1(the medical tube piece) is greater than that of the deformed side bellows tube portions included in the medical tube 1(the medical tube piece). The medical tube 1 (medical tube piece 10 to be described below) according to the first modification includes more reference rings 20 than deformed rings 300.

As shown in FIG. 5(B), assuming the above-described configuration as a medical tube piece 10, a medical tube 1 including a series of medical tube pieces 10 is also encompassed by the scope of the present invention. If medical tube pieces 10 are connected in series, a connection portion 31C where tube end portions 31B and 31A are connected to each other is formed between adjoining medical tube pieces 10. Connection portions 31C may also be formed between subsequent adjoining medical tube pieces 10. Such a connection portion 31C is a kind of deformed side tube portion 3.

As shown in FIG. 5(B), the tube end portions 31A and 31B have an axial length less than that of the medical tube piece 10, desirably less than or equal to 1/5 that of the medical tube piece 10. As shown in FIGS. 5(A) and 5(B), the medical tube piece 10 may include both (see FIGS. 5(A) and 5(B)) or either one of a reference side bellows tube portion 2 having an axial length less than that of the tube end portions 31A and 31B (for example, the first reference side bellows tube portion 2A) and a reference side bellows tube portion 2 having an axial length greater than that of the tube end portions 31A and 31B (for example, the second reference side bellows tube portion 2B and the third reference side bellows tube portion 2C). The medical tube piece 10 may also include either one (see FIGS. 5(C) and 5(D)) or both (see FIGS. 5(A) and 5(B)) of a deformed side bellows tube portion 30 having an axial length less than that of the tube end portions 31A and 31B (for example, the second deformed side bellows tube portion 30B) and a deformed side bellows tube portion 30 having an axial length greater than that of the tube end portions 31A and 31B (for example, the first deformed side bellows tube portion 30A) .

In an overall view of the medical tube 1 including a series of medical tube pieces 10, a first deformed side bellows tube portion 30A including a series of four deformed rings 300, a second deformed side bellows tube portion 30B including a series of three deformed rings 300, and a connection portion 31C having a straight tube shape are repeatedly arranged in this order along the axial direction L with reference side bellows tube portions 2 therebetween.

FIG. 5(C) shows a medical tube 1 according to a second modification. This medical tube 1 includes a tube end portion 31A, a first reference side bellows tube portion 2A, a first deformed side bellows tube portion 30A, a second reference side bellows tube portion 2B, and a tube end portion 31B connected in order. In the medical tube 1 according to the second modification, both the number of reference side bellows tube portions 2 and the number of deformed side tube portions 3 are one less than in the medical tube 1 according to the first modification.

The first reference side bellows tube portion 2A and the second reference side bellows tube portion 2B have respective different axial lengths. In other words, the first reference side bellows tube portion 2A and the second reference side bellows tube portion 2B include series of respective different numbers of reference rings 20. In the medical tube 1 shown in FIG. 5(C), the first reference side bellows tube portion 2A includes a series of 11 reference rings 20, and the second reference side bellows tube portion 2B includes a series of 10 reference rings 20. As a result, the axial length of the first reference side bellows tube portion 2A is greater than that of the second reference side bellows tube portion 2B.

In the medical tube 1 shown in FIG. 5(C), the first deformed side bellows tube portion 30A includes a series of two deformed rings 300. The first deformed side bellows tube portion 30A has an axial length less than those of both the first reference side bellows tube portion 2A and the second reference side bellows tube portion 2B. The first deformed side bellows tube portion 30A may be regarded as being located substantially in the center of the medical tube 1 according to the second modification.

The total of the axial lengths of the first reference side bellows tube portion 2A and the second reference side bellows tube portion 2B is greater than the axial length of the first deformed side bellows tube portion 30A. In other words, A sum of an axial length of the reference side bellows tube portions included in the medical tube 1(the medical tube piece) is greater than that of the deformed side bellows tube portions included in the medical tube 1(the medical tube piece). The medical tube 1 (medical tube piece 10) according to the second modification includes more reference rings 20 than deformed rings 300.

As shown in FIG. 5(D), assuming the configuration of the second modification as a medical tube piece 10, a medical tube 1 including a series of medical tube pieces 10 is also encompassed by the scope of the present invention. If medical tube pieces 10 are connected in series, a connection portion 31C where tube end portions 31B and 31A are connected to each other is formed between adjoining medical tube pieces 10. Connection portions 31C may also be formed between subsequent adjoining medical tube pieces 10. Such a connection portion 31C is a kind of deformed side tube portion 3.

In an overall view of the medical tube 1 including a series of medical tube pieces 10, a first reference side bellows tube portion 2A, a first deformed side bellows tube portion 30A, a second reference side bellows tube portion 2B, and a connection portion 31C are repeatedly arranged in this order along the axial direction L.

The medical tube 1 is not limited to the configurations of the above-described modifications, and may have any configuration where reference side bellows tube portions 2 and deformed side bellows tube portions 30 are alternately arranged in series. Applicable configurations of the medical tube 1 may also include ones where at least some of the deformed side bellows tube portions 30 are replaced with a single deformed ring 300.

### <Axial Lengths of Medical Pipes According to Modifications of First Embodiment>

In the first modification of the first embodiment, the tube end portion 31A has an axial length of 19 mm. The first reference side bellows tube portion 2A has an axial length of 9.7 mm. The first deformed side bellows tube portion 30A has an axial length of 20 mm. The second reference side bellows tube portion 2B has an axial length of 45 mm. The second deformed side bellows tube portion 30B has an axial length of 15 mm. The third reference side bellows tube portion 2C has an axial length of 25 mm. The tube end portion 31B has an axial length of 19 mm.

In the second modification of the first embodiment, the tube end portion 31A has an axial length of 19 mm. The first reference side bellows tube portion 2A has an axial length of 54.7 mm. The first deformed side bellows tube portion 30A has an axial length of 10 mm. The second reference side bellows tube portion 2B has an axial length of 50 mm. The tube end portion 31B has an axial length of 19 mm.

In the first and second modifications of the first embodiment, the reference rings 20 and the deformed rings 300 have an axial length of 5 mm. Note that the reference ring 20 continuous with the tube end portion 31A has an axial length of 4.7 mm. The reference ridge shape of the reference rings 20 has a ridge height of 3.5 mm. The deformed ridge shape of the deformed rings 300 has a ridge height of 2.7 mm. The diameter of the imaginary reference cylindrical surface S (the inner diameter of the medical tube 1) is 22 mm.

In the first embodiment, the axial lengths of the reference side bellows tube portions 2 (first reference side bellows tube portion 2A and second reference side bellows tube portion 2B) are less than 112 mm. However, the medical tube 1 may include a reference side bellows tube portion 2 having an axial length of 112 mm or more.

As described above, in the present embodiment including the first and second modifications, the inner wall-side ridge height H2 of the deformed ridge shapes of the deformed rings 300 is less than the inner wall-side ridge height H1 of the reference ridge shapes of the reference rings 20. The bases 304 of the deformed ridge shapes of the deformed rings 300, the bases 24 of the reference ridge shapes of the reference rings 20, and the innermost parts of the tube end portions 31 closest to the center axis 11 of the medical tube 1 in the radial direction R (the base 310AA of the deformed ring 310A, the inner wall surface 310BA of the deformed ring 310B, the two bases 310CA of the deformed ring 310C, and the inner wall surface 310DA of the deformed ring 310D) are located on the imaginary reference cylindrical surface S. As a result, in the present embodiment including the first and second modifications, the medical tube 1 has an air pathway 40, first internal spaces 25 (see FIGS. 2(A) and 2(B)) and second internal spaces 35 (see FIGS. 2(A) and 2(B)). The air pathway 40 is configured with the imaginary reference cylindrical surface S, which is located at the minimum inner diameter of the medical tube 1, as a boundary(the minimum inner diameter part of the medical tube 1). The first internal spaces 25 are the space between an inner wall surface of the reference side bellows tube portions 2 and the boundary of the air pathway 40(the minimum inner diameter part of the medical tube 1) and is surrounded by both. The reference side bellows tube portions 2 is located outside of the boundary of the air pathway 40 in the radial direction R. The second internal spaces 35 are the space between an inner wall surface of the deformed side tube portions 3 and the boundary of the air pathway 40 and is surrounded by both. The deformed side tube portions 3 is located outside of the boundary of the air pathway 40 in the radial direction R. There is no obstacle to the passing gas in the space surrounded by the air pathway 40 inside the imaginary reference cylindrical surface S. As shown in FIGS. 2(A) and 2(B), the second internal spaces 35 include ones corresponding to the deformed side bellows tube portions 30 and ones corresponding to the tube end portions 31. The second internal spaces 35 have a smaller height in the radial direction R than the first internal spaces 25. Moreover, the total axial length of the reference side bellows tube portions 2 in each interval of the medical tube 1 (medical tube piece 10) is greater than or equal to that of the deformed side bellows tube portions 30 in each interval. Or equivalently, the total number of reference rings 20 in the medical tube 1 (medical tube piece 10) is greater than that of deformed rings 300.

### <Second Embodiment>

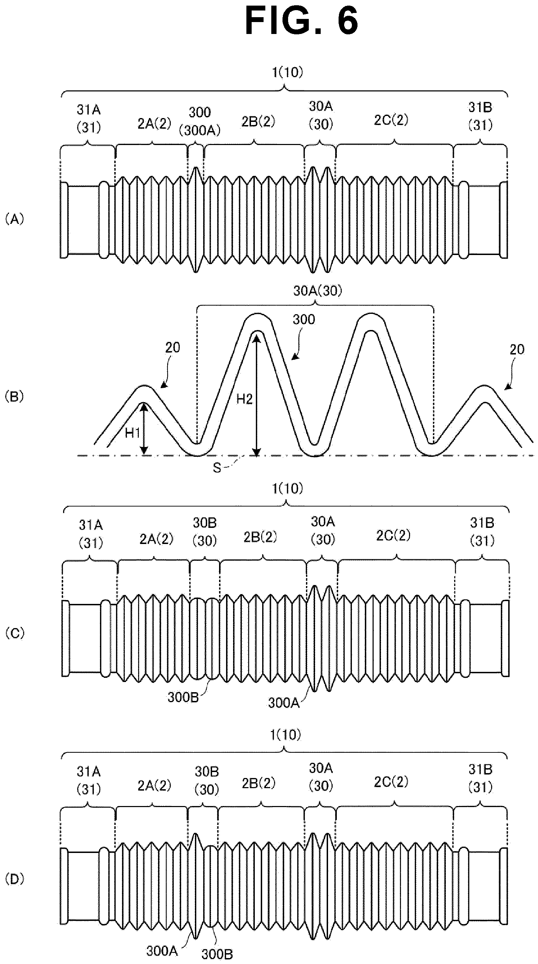

A medical tube 1 according to a second embodiment of the present invention will be described with reference to FIGS. 6(A) to 6(D). Unlike the medical tube 1 according to the first embodiment, the medical tube 1 according to the second embodiment is configured so that, as shown in FIG. 6(B), the inner wall-side ridge height H2 of the deformed ridge shape of deformed ridges 300 is greater than the inner wall-side ridge height H1 of reference ridge shape of the reference ridges 20. FIG. 6(A) shows an example of the medical tube 1 including such deformed rings 300. This medical tube 1 includes a tube end portion 31A, a first reference side bellows tube portion 2A, a single deformed ring 300, a second reference side bellows tube portion 2B, a first deformed side bellows tube portion 30A, a third reference side bellows tube portion 2C, and a tube end portion 31B connected in order.

The first reference side bellows tube portion 2A according to the second embodiment includes a series of five reference rings 20. The second reference side bellows tube portion 2B includes a series of seven reference rings 20. The third reference side bellows tube portion 2C includes a series of eight reference rings 20. As a result, the first reference side bellows tube portion 2A, the second reference side bellows tube portion 2B, and the third reference side bellows tube portion 2C increase in axial length in this order. The first deformed side bellows tube portion 30A includes a series of two deformed rings 300. The axial lengths of the first reference side bellows tube portion 2A, the second reference side bellows tube portion 2B, and the third reference side bellows tube portion 2C are greater than those of the single deformed ring 300 and the first deformed side bellows tube portion 30A. In the second embodiment, the single deformed ring 300 and the first deformed side bellows tube portion 30A can be regarded as located on both sides of the center of the medical tube 1. The first deformed side bellows tube portion 30A is closer to the center of the medical tube 1 than the single deformed ring 300.

The medical tube 1 may include a deformed ring 300A of which the inner wall-side ridge height H2 of the deformed ridge shape is greater than the inner wall-side ridge height H1 of the reference ridge shape of the reference rings 20 and a deformed ring 300B of which the inner wall-side ridge height H2 is less than the inner wall-side ridge height H1. FIG. 6(C) shows an example of such a medical tube 1, where the single deformed ring 300 of the medical tube 1 according to the second embodiment is replaced with a second deformed side bellows tube portion 30B including a plurality of deformed rings 300B in series. FIG. 6(D) shows another example of such a medical tube 1, where the single deformed ring 300 of the medical tube 1 according to the second embodiment is replaced with a second deformed side bellows tube portion 30B including a series of deformed rings 300A and 300B. The replacing second deformed side bellows tube portion 30B may include a plurality of deformed rings 300A and 300B alternately arranged in series, or may partly include a plurality of deformed rings 300A in series and/or a plurality of deformed rings 300B in series. Any of such configurations is encompassed by the scope of the present invention.

### <Axial Lengths of Medical Pipe According to Second Embodiment>

In the second embodiment shown in FIGS. 6(A) to 6(D), the tube end portion 31A has an axial length of 19 mm. The first reference side bellows tube portion 2A has an axial length of 24.7 mm. The single deformed ring 300(300A) has an axial length of 5 mm. The second reference side bellows tube portion 2B has an axial length of 35 mm. The first deformed side bellows tube portion 30A has an axial length of 10 mm. The third reference side bellows tube portion 2C has an axial length of 40 mm. The tube end portion 31B has an axial length of 19 mm.

In the second embodiment, the reference rings 20 and the deformed rings 300(300A, 300B) have an axial length of 5 mm. Note that the reference ring 20 continuous with the tube end portion 31A has an axial length of 4.7 mm. The reference ridge shape of the reference rings 20 has a ridge height of 3.5 mm. The deformed ridge shape of the deformed rings 300A has a ridge height of 7.0 mm. The diameter of the imaginary reference cylindrical surface S (the inner diameter of the medical tube 1) is 22 mm.

In the second embodiment, the axial lengths of the reference side bellows tube portions 2 (first reference side bellows tube portion 2A, second reference side bellows tube portion 2B, and third reference side bellows tube portion 2C) are less than 112 mm. However, the medical tube 1 may include a reference side bellows tube portion 2 having an axial length of 112 mm or more.

### <Third Embodiment>

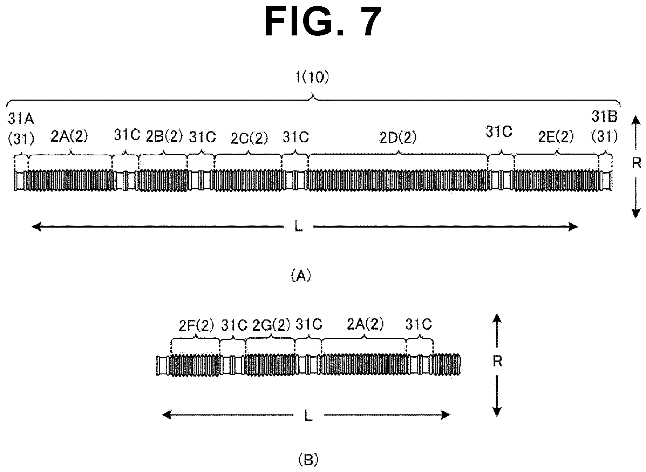

A medical tube 1 according to a third embodiment of the present invention will be described with reference to FIGS. 7(A) and 7(B). The medical tube 1 according to the third embodiment includes reference side bellows tube portions 2 and connection portions 31C alternately connected. The connection portions 31C are a kind of deformed side tube portions. Specifically, as shown in FIG. 7(A), the medical tube 1 according to the third embodiment is formed by connecting a tube end portion 31A that is a kind of deformed side tube portion, a first reference side bellows tube portion 2A, a connection portion 31C that is a kind of deformed side tube portion, a second reference side bellows tube portion 2B, a connection portion 31C that is a kind of deformed side tube portion, a third reference side bellows tube portion 2C, a connection portion 31C that is a kind of deformed side tube portion, a fourth reference side bellows tube portion 2D, a connection portion 31C that is a kind of deformed side tube portion, a fifth reference side bellows tube portion 2E, and a tube end portion 31B that is a kind of deformed side tube portion in this order.

The first reference side bellows tube portion 2A, the second reference side bellows tube portion 2B, the third reference side bellows tube portion 2C, the fourth reference side bellows tube portion 2D, and the fifth reference side bellows tube portion 2E include series of different numbers of reference rings 20. In the medical tube 1 shown in FIG. 7(A), the first reference side bellows tube portion 2A includes a series of 23 reference rings 20. The second reference side bellows tube portion 2B includes a series of 13 reference rings 20. The third reference side bellows tube portion 2C includes a series of 18 reference rings 20. The fourth reference side bellows tube portion 2D includes a series of 39 reference rings 20. The fifth reference side bellows tube portion 2E includes a series of 23 reference rings 20. As a result, in the interval from the first reference side bellows tube portion 2A to the second reference side bellows tube portion 2B, the axial length of a reference side bellows tube portion 2 decreases. In the interval from the second reference side bellows tube portion 2B to the fourth reference side bellows tube portion 2D, the axial length of a reference side bellows tube portion 2 increases from the second reference side bellows tube portion 2B to the fourth reference side bellows tube portion 2D. In the interval from the fourth reference side bellows tube portion 2D to the fifth reference side bellows tube portion 2E, the axial length of a reference side bellows tube portion 2 decreases.

The numbers of the reference rings 20 in the respective series are just examples and may be different. The reference side bellows tube portions 2 (first reference side bellows tube portion 2A, second reference side bellows tube portion 2B, third reference side bellows tube portion 2C, fourth reference side bellows tube portion 2D, and fifth reference side bellows tube portion 2E) may have an interval where ones having a large axial length and ones a small axial length are alternately arranged via the connection portions 31C. The reference side bellows tube portions 2 may have an interval where the reference side bellows tube portions 2 are arranged to increase or decrease in axial length toward one side in the axial direction. As shown in FIG. 7(B), the reference side bellows tube portions 2 may have an interval where the axial length is constant. In FIG. 7(B), two reference side bellows tube portion 2 (sixth reference side bellows tube portion 2F and seventh reference side bellows tube portion 2G) connected via connection 31C are added between the first reference side bellows tube portion 2A and the tube end portion 31A in FIG. 7(A). The two reference side bellows tube sections 2 are similar to the second reference side bellows tube portion 2B.

In the third embodiment, the axial lengths of the first reference side bellows tube portion 2A, the second reference side bellows tube portion 2B, the third reference side bellows tube portion 2C, the fourth reference side bellows tube portion 2D, the fifth reference side bellows tube portion 2E, the sixth reference side bellows tube portion 2F, and the seventh reference side bellows tube portion 2G are greater than that of the connection portions 31C that are a kind of deformed side tube portions. However, this is not restrictive, and a reference side bellows tube portion 2 having an axial length less than that of the connection portions 31C may be included. However, the total axial length of the reference side bellows tube portions 2 in each interval of the medical tube 1 according to the third embodiment is desirably greater than that of the connection portions 31C in each interval.

### <Axial Lengths of Medical Pipe According to Third Embodiment>

In the third embodiment, the tube end portion 31A has an axial length of 19 mm. The first reference side bellows tube portion 2A has an axial length of 115 mm. The second reference side bellows tube portion 2B has an axial length of 62 mm. The third reference side bellows tube portion 2C has an axial length of 92 mm. The fourth reference side bellows tube portion 2D has an axial length of 194 mm. The fifth reference side bellows tube portion 2E has an axial length of 115 mm. The sixth reference side bellows tube portion 2F has an axial length of 62 mm. The seventh reference side bellows tube portion 2G has an axial length of 62 mm. The tube end portion 31B has an axial length of 19 mm.

Again, in the third embodiment, the reference rings 20 and the deformed rings 300 have an axial length of 5 mm. Note that the reference ring 20 continuous with the tube end portion 31A has an axial length of 4.7 mm. The reference ridge shape of the reference rings 20 has a ridge height of 3.5 mm. The diameter of the imaginary reference cylindrical surface S (the inner diameter of the medical tube 1) is 22 mm.

In the third embodiment, the reference side bellows tube portions 2 (first reference side bellows tube portion 2A, second reference side bellows tube portion 2B, third reference side bellows tube portion 2C, fourth reference side bellows tube portion 2D, fifth reference side bellows tube portion 2E, sixth reference side bellows tube portion 2F, and seventh reference side bellows tube portion 2G) include ones having an axial length less than 112 mm and ones having an axial length greater than 112 mm. However, all the reference side bellows tube portions 2 (first reference side bellows tube portion 2A, second reference side bellows tube portion 2B, third reference side bellows tube portion 2C, fourth reference side bellows tube portion 2D, fifth reference side bellows tube portion 2E, sixth reference side bellows tube portion 2F, and seventh reference side bellows tube portion 2G) may have an axial length less than 112 mm.

### <Fourth Embodiment>

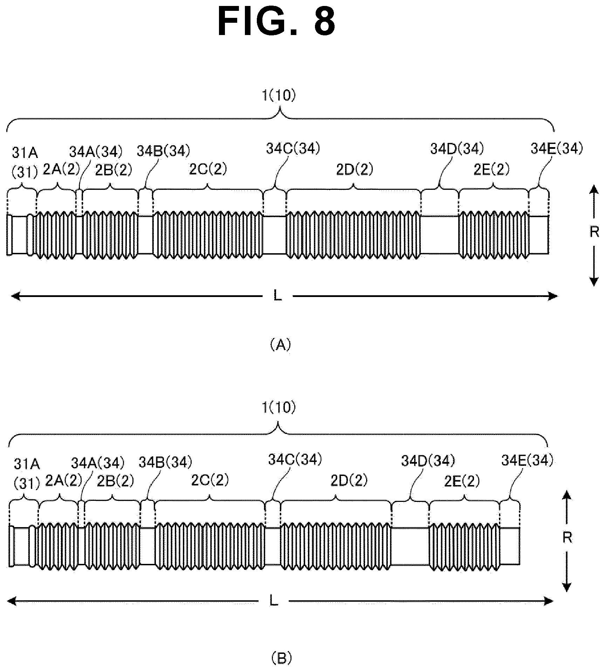

A medical tube 1 according to a fourth embodiment of the present invention will be described with reference to FIGS. 8(A) and 8(B). The medical tube 1 according to the fourth embodiment includes deformed side tube portions 3 configured by cylindrical portions 34. Reference side bellows tube portions 2 and the cylindrical portions 34 are alternately connected. The plurality of cylindrical portions 34 have respective different axial lengths. In order to distinguish them from one another, the cylindrical portions 34 will be referred to, in order from the left in FIG. 8(A), as a first cylindrical portion 34A, a second cylindrical portion 34B, a third cylindrical portion 34C, a fourth cylindrical portion 34D, and a fifth cylindrical portion 34E.

Specifically, the medical tube 1 according to the fourth embodiment includes, for example, a tube end portion 31A, a first reference side bellows tube portion 2A, the first cylindrical portion 34A, a second reference side bellows tube portion 2B, the second cylindrical portion 34B, a third reference side bellows tube portion 2C, the third cylindrical portion 34C, a fourth reference side bellows tube portion 2D, the fourth cylindrical portion 34D, a fifth reference side bellows tube portion 2E, and the fifth cylindrical portion 34E connected in order.

As shown in FIG. 8(A), the first reference side bellows tube portion 2A according to the fourth embodiment includes a series of five reference rings 20. The second reference side bellows tube portion 2B includes a series of seven reference rings 20. The third reference side bellows tube portion 2C includes a series of 14 reference rings 20. The fourth reference side bellows tube portion 2D includes a series of 17 reference rings 20. The fifth reference side bellows tube portion 2E includes a series of nine reference rings 20. As a result, in the interval from the first reference side bellows tube portion 2A to the fourth reference side bellows tube portion 2D, the axial length of a reference side bellows tube portion 2 increases from the first reference side bellows tube portion 2A toward the fourth reference side bellows tube portion 2D. In the interval from the fourth reference side bellows tube portion 2D to the fifth reference side bellows tube portion 2E, the axial length of a reference side bellows tube portion 2 decreases from the fourth reference side bellows tube portion 2D toward the fifth reference side bellows tube portion 2E.

Similarly, as shown in FIG. 8(A), in the interval from the first cylindrical portion 34A to the fourth cylindrical portion 34D, the axial length of a cylindrical portion increases from the first cylindrical portion 34A toward the fourth cylindrical portion 34D. In the interval from the fourth cylindrical portion 34D to the fifth cylindrical portion 34E, the axial length of a cylindrical portion decreases from the fourth cylindrical portion 34D toward the fifth cylindrical portion 34E.

Again, in the fourth embodiment, the numbers of reference rigs 20 in the respective series are just examples, and may be different. The medical tube 1 according to the fourth embodiment may have an interval where the reference side bellows tube portions 2 having a large axial length and the reference side bellows tube portions 2 having a small axial length are alternately arranged via the cylindrical portions 34 (first cylindrical portion 34A, second cylindrical portion 34B, third cylindrical portion 34C, and fourth cylindrical portion 34D). The medical tube 1 according to the fourth embodiment may have an interval where the reference side bellows tube portions 2 are arranged to increase or decrease in axial length toward one side in the axial direction. As shown in FIG. 8(B), the medical tube 1 according to the fourth embodiment may have an interval where the axial length of the reference side bellows tube portions 2 is constant (for example, the interval including the third reference side bellows tube portion 2C and the fourth reference side bellows tube portion 2D). The same can apply to the plurality of cylindrical portions 34.

In the fourth embodiment, the axial lengths of the first reference side bellows tube portion 2A, the second reference side bellows tube portion 2B, the third reference side bellows tube portion 2C, the fourth reference side bellows tube portion 2D, and the fifth reference side bellows tube portion 2E are greater than those of the cylindrical portions 34 (first cylindrical portion 34A, second cylindrical portion 34B, third cylindrical portion 34C, fourth cylindrical portion 34D, and fifth cylindrical portion 34E). However, this is not restrictive, and a reference side bellows tube portion 2 having an axial length less than that of a cylindrical portion 34 may be included. However, the total axial length of the reference side bellows tube portions 2 in each interval of the medical tube 1 according to the fourth embodiment is desirably greater than that of the cylindrical portions 34 in each interval.

### <Axial Lengths of Medical Pipe According to Fourth Embodiment>

In the fourth embodiment, the tube end portion 31A has an axial length of 19 mm. The first reference side bellows tube portion 2A has an axial length of 24.7 mm. The first cylindrical portion 34A has an axial length of 5 mm. The second reference side bellows tube portion 2B has an axial length of 35 mm. The second cylindrical portion 34B has an axial length of 10 mm. The third reference side bellows tube portion 2C has an axial length of 70 mm. The third cylindrical portion 34C has an axial length of 15 mm. The fourth reference side bellows tube portion 2D has an axial length of 85 mm. The fourth cylindrical portion 34D has an axial length of 25 mm. The fifth reference side bellows tube portion 2E has an axial length of 45 mm. The fifth cylindrical portion 34E has an axial length of 12 mm.

Again, in the fourth embodiment, the reference rings 20 have an axial length of 5 mm. Note that the reference ring 20 continuous with the tube end portion 31A has an axial length of 4.7 mm. The reference ridge shape of the reference rings 20 has a ridge height of 3.5 mm. The diameter of the imaginary reference cylindrical surface S (the inner diameter of the medical tube 1) is 22 mm.

In the fourth embodiment, the axial lengths of the reference side bellows tube portions 2 (first reference side bellows tube portion 2A, second reference side bellows tube portion 2B, third reference side bellows tube portion 2C, fourth reference side bellows tube portion 2D, and fifth side bellows tube portion 2E) are less than 112 mm. However, the medical tube 1 may include a reference side bellows tube portion 2 having an axial length of 112 mm or more.

### <Modifications of Fourth Embodiment>

Modifications of the medical tube 1 according to the fourth embodiment will be described with reference to FIGS. 9(A) to 9(E). FIG. 9(A) shows a medical tube 1 according to a first modification. As shown in FIG. 9(A), this medical tube 1 is different from the medical tube 1 according to the fourth embodiment in the portions corresponding to the third cylindrical portion 34C and the fourth cylindrical portion 34D. The rest of the structure is the same.

The third cylindrical portion 34C of the medical tube 1 according to the first modification has a peripheral wall 34CA that is formed to protrude radially outward. Specifically, as shown in FIG. 9(B), the third cylindrical portion 34C curves to protrude outward in the radial direction of the third cylindrical portion 34C with respect to the imaginary reference cylindrical surface S. The midsection of the peripheral wall 34CA in the axial direction of the third cylindrical portion 34C is the top of the convex shape.

The fourth cylindrical portion 34D of the medical tube 1 according to the first modification has a peripheral wall 34DA that is formed concavo-convex in the axial direction of the medical tube 1. Specifically, as shown in FIG. 9(C), a midsection 340 of the peripheral wall 34DA in the axial direction of the fourth cylindrical portion 34D forms substantially the same surface as the imaginary reference cylindrical surface S.

A part of the peripheral wall 34DA continuous on one side of the axial direction at the midsection of the peripheral wall 34DA in the axial direction of the fourth cylindrical portion 34D is recessed inward in the radial direction of the fourth cylindrical portion 34D with respect to the imaginary reference cylindrical surface S, whereby a recess 341 is formed. Another part of the peripheral wall 34DA continuous on other side of the axial direction at the midsection of the peripheral wall 34DA in the axial direction of the fourth cylindrical portion 34D is also recessed inward in the radial direction of the fourth cylindrical portion 34D with respect to the imagery reference cylindrical surface S, whereby a recess 342 is formed. The recess 341 is deeper than the recess 342.

FIGS. 9(D) and 9(E) show a medical tube 1 according to a second modification of the fourth embodiment. This medical tube 1 is formed by replacing the peripheral wall in the middle of the fourth reference side bellows tube portion 2D of the medical tube 1 according to the fourth embodiment with a sixth cylindrical portion 34F. The sixth cylindrical portion 34F has a recessed shape, being curved and recessed radially inward with respect to the imaginary reference cylindrical surface S. Such a configuration is also encompassed by the scope of the present invention.

The concavo-convex configurations of the peripheral walls of the cylindrical portions are not limited to the above-described configurations. Configurations with a different number of recesses, a different number of protrusions, and/or a different pattern of recesses and protrusions are also encompassed by the scope of the present invention. Assuming the configuration shown in FIG. 9(A) or 9(D) as a medical tube piece 10, a medical tube 1 including a series of medical tube pieces 10 is also encompassed by the scope of the present invention.

### <Axial Lengths of Medical Pipes According to Modifications of Fourth Embodiment>

In the first modification of the fourth embodiment, the third cylindrical portion 34C has an axial length of 15.43 mm. The fourth cylindrical portion 34D has an axial length of 19.9 mm. The other portions have the same axial lengths as those of the medical tube 1 according to the fourth embodiment. In the second modification of the fourth embodiment, the sixth cylindrical portion 34F has an axial length of 5 mm. The other portions have the same axial lengths as those of the medical tube 1 according to the fourth embodiment.

In the first and second modifications, the reference rings 20 have an axial length of 5 mm. Note that the reference ring 20 continuous with the tube end portion 31A has an axial length of 4.7 mm. The reference ridge shape of the reference rings 20 has a ridge height of 3.5 mm. The diameter of the imaginary reference cylindrical surface S (the inner diameter of the medical tube 1) is 22 mm.

In the first and second modifications, the axial lengths of the reference side bellows tube portions 2 (first reference side bellows tube portion 2A, second reference side bellows tube portion 2B, third reference side bellows tube portion 2C, fourth reference side bellows tube portion 2D, and fifth reference side bellows tube portion 2E) are less than 112 mm. However, the medical tube 1 may include a reference side bellows tube portion 2 having an axial length of 112 mm or more.

In the medical tubes 1 according to the first to fourth embodiments configured to be described above, reference side bellows tube portions 2 and deformed side tube portions 3 (deformed side bellows tube portions 30 and tube end portions 31) having various axial lengths are disposed interval by interval to reduce intervals where the same shapes are redundantly repeated. As will be described below, acoustic power levels inside the medical tubes 1 can thus be reduced compared to that of a conventional medical tube, even when air is supplied into the medical tubes 1 according to the modifications of the embodiments. This enables noise reduction of the medical tubes 1.

### <Examples>

The inventor generated medical tubes 1A to 1H having an axial length of approximate 1 m based on the medical tubes 1 according to the above-described first to fourth embodiments and a medical tube 1I serving as a comparative example by using predetermined simulation software. The inventor then conducted a simulation of the audio power levels (dB) inside the medical tubes 1 for the case of passing gas through the medical tubes 1A to 1I at 150 L/min per unit time using the simulation software.

The medical tube 1A included a series of three medical tube pieces 10 assumed in the first embodiment. Note that a single tube end portion 31 was formed at each junction of the medical tube pieces 10 instead of a connection portion 31C. The medical tube 1B included a series of seven medical tube piece 10 assumed in the first modification of the first embodiment. The medical tube 1C included a series of seven medical tube pieces 10 assumed in the second modification of the first embodiment. The medical tube 1D as shown in FIG. 7(B), includes the reference side bellows tube sections 2F and 2G added to what is regarded as the medical tube piece 10 in the third embodiment shown in FIG.7(A). The medical tube 1E included a series of three medical tube pieces 10 assumed in the fourth embodiment (see FIG. 8(A)). The medical tube 1F included a series of seven medical tube pieces 10 assumed in the second embodiment (see FIG. 6(A)). The medical tube 1G included a series of three medical tube pieces 10 assumed in the first modification of the fourth embodiment (see FIG. 9(A)). The medical tube 1H included a series of three medical tube pieces 10 assumed in the second modification of the fourth embodiment (see FIG. 9(D)) where one of the reference rings in the fifth reference side bellows tube portion 2E was replaced with a deformed ring having an inner wall-side ridge height greater than that of the reference ring. The medical tube 1I was prepared as a comparative example and included a series of seven medical tube pieces 10A assumed and shown in FIG. 4(B). The connection portions 31C were formed at the junctions of the medical tube pieces 10 except for the medical tubes 1A, 1E, 1G, and 1H. The medical tubes 1A to 1I had the same axial lengths and sizes as described in the above-described embodiments.

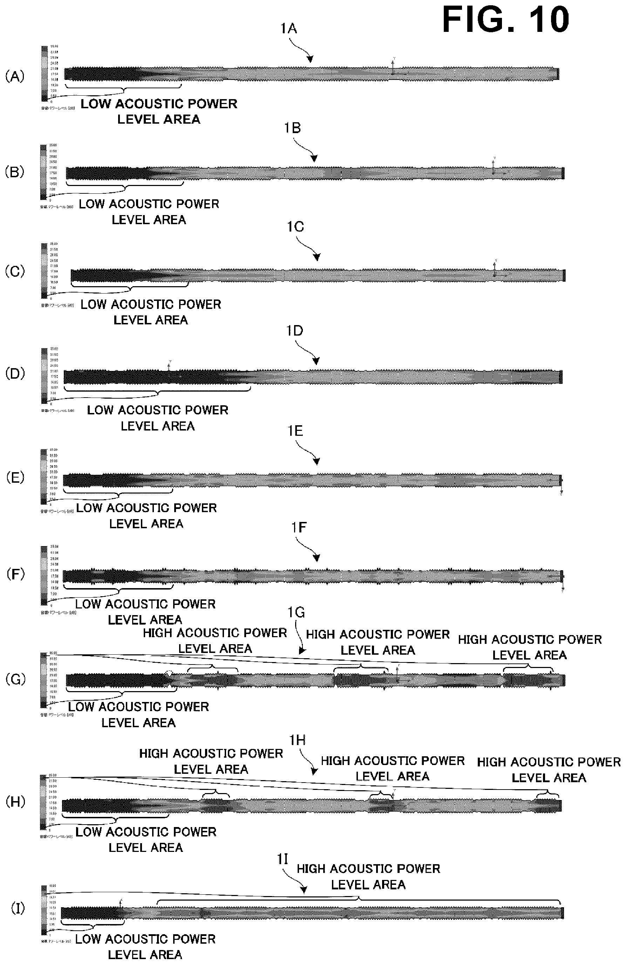

FIGS. 10(A) to 10(I) show the simulation results. The acoustic power levels (dB) inside the medical tubes 1A to 1G are shown by color densities. The dark-colored areas extending from the left ends of the medical tubes 1A to 1I shown in FIGS. 10(A) to 10(I) represent where the acoustic power level (dB) was as low as 0 to 3.5 dB. Such areas will be referred to as low acoustic power level areas. By contrast, the three dark-colored areas on the right of the low acoustic power level areas of the medical tubes 1G and 1H shown in FIGS. 10(G) and 10(H) represent where the acoustic power level (dB) was as high as 31.5 to 35.0 dB. Such areas will be referred to as high acoustic power level areas. The high acoustic power level area shown in FIG. 10(I) represents where the acoustic power level (dB) was as high as 34.14 to 39.01 dB.

FIG. 11 is a table showing evaluations on the acoustics inside the medical tubes based on the simulation results. The evaluations in the table shown in FIG. 11 take into account not only the magnitudes of the acoustic power levels but also the manner in which the acoustic power levels change. The evaluations "S", "A", "B", "C", and "F" in the table shown in FIG. 11 are arranged in order from the one with the highest evaluation.

As shown in FIG. 10(I), the medical tube 1I showed a low acoustic power level area at the left end where the axial length is short compared to the other medical tubes 1A to 1G. All the rest was a high acoustic power level area. The medical tube 1I thus had a high acoustic power level and produced a noticeable level of noise in most of the intervals. In the table shown in FIG. 11, the acoustic evaluation on the medical tube 1I is therefore "F".

As shown in FIG. 10(C), the medical tube 1C changed less in color (acoustic power level) in the area on the right of the low acoustic power level area than the other medical tubes. The entire acoustic power level can thus be said to be uniform. Supplying gas through the medical tube 1C thus produces a less noticeable level of noise than with the other medical tubes 1A, 1B, 1D to 1I. In the table shown in FIG. 11, the acoustic evaluation on the medical tube 1C is therefore "S".

The medical tubes 1A, 1B, and 1D changed more in color (acoustic power level) than the medical tube 1C but less than the medical tubes 1E to 1H. Fewer changes in the acoustic power level make the noise less noticeable. Supplying gas through the medical tubes 1A, 1B, and 1D therefore produces a less noticeable level of noise than with the medical tubes 1E to 1I. In the table shown in FIG. 11, the acoustic evaluations on the medical tubes 1A, 1B, and 1D are therefore "A".

As shown in FIGS. 10(E) and 10(F), the medical tubes 1E and 1F showed areas of somewhat dark color (somewhat high acoustic power level) distributed all over. Supplying gas through the medical tubes 1E and 1F thus produces a more noticeable level of noise than with the medical tubes 1A to 1D but less than with the medical tubes 1G to 1I. In the table shown in FIG. 11, the acoustic evaluations on the medical tubes 1E and 1F are therefore "B".

As shown in FIGS. 10(G) and 10 (H), the medical tubes 1G and 1H showed three high acoustic power level areas at predetermined distances. A lot of color-changing areas were also seen between the high acoustic power level areas. Supplying gas through the medical tubes 1G and 1H thus produces a more noticeable level of noise than with the medical tubes 1A to 1F but less than with the medical tube 1I. In the table shown in FIG. 11, the acoustic evaluations on the medical tubes 1G and 1H are therefore "C", which is lower than "B" and higher than "F".

As shown in FIGS. 10(G) and 10(H), the high acoustic power level areas of both the medical tubes 1G and 1H extended from the areas where the peripheral walls of the cylindrical portions were recessed inward with respect to the imaginary reference cylindrical surface S (see the recess 341 in the peripheral wall 34CA in FIG. 9(B) and the sixth cylindrical portion 34F of recessed shape in FIG. 9(D)) or their vicinities. This suggests that the acoustic power level tends to increase at portions where the diameter of the medical tube 1 is smaller than that of the imaginary reference cylindrical surface S.

The result of the simulation conducted on the medical tube 1I using the above-described simulation software showed that the high acoustic power level area spread over the wide range in the medical tube 1I. The inventor has considered that it is ascribable to the lack of changes in the unit shapes repeated in the medical tube 1I (hereinafter, referred to as repeating unit shapes) and the redundant repetition of the repeating unit shapes. The inventor has then contrived to change the repeating unit shapes of the medical tube with respect to the medical tube 1I and/or change the axial length interval by interval instead of simple repetitions. From the above-described simulation results, it can be seen that such contrivances successfully reduce the noise level of the medical tubes. Specifically, the noise level was successfully reduced compared to that of the medical tube 1I by replacing part of the medical tube 1I with deformed rings 300, 300A, and/or 300B to make the repeating unit shapes more complicated (medical tubes 1A to 1C and 1F). In particular, the noise level was more successfully reduced by replacing part of the medical tube 1I with deformed rings 300B having a ridge height smaller than the inner wall-side ridge height of the reference ridge shape of the reference rings 20 (medical tubes 1A to 1C). The noise level was also successfully reduced compared to that of the medical tube 1I by configuring the medical tube so that the repeating unit shapes of the medical tube 1I had various axial lengths (medical tube 1D). The noise level was also successfully reduced compared to that of the medical tube 1I by configuring the medical tube so that the reference side bellows tube portions 2 and the cylindrical portions 34 have different axial lengths interval by interval (medical tubes 1E, 1G, and 1H).

The medical tube 1I has six connection portions 31C serving as locations to be cut for length adjustment. Cutting a connection portion 31C halfway produces tube end portions 31A and 31B at the section. Reducing the number of connection portions 31C makes fine adjustments in length difficult. The reference side bellows tube portions 2 of the medical tube 1I are therefore difficult to increase in length. For such a reason, the total axial length of the tube end portions 31A and 31B and the reference side bellows tube portion 2 is typically set to 150 mm, and the axial length of the reference side bellows tube portion 2 is typically set to 112 mm. According to the present invention, the noise level in a medical tube 1 can be reduced by contriving the repeating unit shapes of the medical tube 1 while including reference side bellows tube portions 2 having an axial length less than 112 mm. Note that a medical tube 1 may include both reference side bellows tube portions 2 having an axial length greater than or equal to 112 mm and ones having an axial length less than 112 mm.

The medical tube 1 according to the present invention is not limited to the above-described embodiments, and various modifications can be made without departing from the gist of the present invention. Thus, various combinations of the components of the medical tubes 1 according to the first to fourth embodiments configured to be described above are naturally encompassed by the scope of the present invention.

### Reference Signs List

- 1: medical tube
- 2: reference side bellows tube portion
- 3: deformed side tube portion
- 10: medical tube piece
- 11: center axis
- 12: opening
- 20: reference ring
- 30: deformed side bellows tube portion
- 31, 31A, 31B: tube end portion
- 31C: connection portion
- 34: cylinder portion
- 300, 300A, 300B, 310A, 310B, 310C, 310D: deformed ring
- 340: midsection
- 341, 342: recess
- 900: inspiratory tube
- 910: unit shape
- 911, 913: tube end portion
- 912: bellows tube portion
- L: axial direction
- R: radial direction
- S: imaginary reference cylindrical surface

A medical tube is provided which can reduce the acoustic power level therein when air is supplied through the tube. The medical tube includes: a reference side bellows tube portion configured to be a flexible bellows-like tube including a plurality of reference rings in series in an axial direction, the reference rings each extending annularly or helically to form a ring; and a deformed side tube portion configured to be a tube shaped differently from the reference side bellows tube portion. Here, a plurality of the reference side bellows tube portions and a plurality of the deformed side tube portions are alternately arranged in the axial direction. At least one of the reference side bellows tube portions has an axial length less than 112 mm.

## Claims

1. A medical tube (1) **characterized by** comprising:
a plurality of reference side bellows tube portions (2) each configured to be a flexible bellows-like tube including a plurality of reference rings (20) in series in an axial direction (L), the reference rings (20) each extending annularly or helically to form a ring;
a plurality of deformed side bellows tube portions (30) each configured to be a tube shaped differently from the reference side bellows tube portion (2), the deformed side bellows tube portions (30) each including a plurality of deformed rings (300) in series in the axial direction (L) to be configured to be a flexible bellows-like tube, the deformed rings (300) each being shaped differently from the reference rings (20) and extending annularly or helically to form a ring; and
a plurality of tube end portions (31,31A,31B) each configured to be a tube shaped differently from the reference side bellows tube portions (2) and including a cylindrical portion (34) having an orthogonal cross section of perfectly circular shape, wherein;
at least one of the reference side bellows tube portions (2) has an axial length greater than those of the deformed side bellows tube (30) portions;
the reference side bellows tube portions and the deformed side bellows tube portions are alternately arranged in the axial direction;
a medical tube piece adjoins the tube end portions (2) at respective ends, where a configuration in which the plurality of reference side bellows tube portions (2) and at least one of the deformed side bellows tube portions (30) are alternately arranged in the axial direction being defined as the medical tube piece (10);
a base of a reference ridge shape and a base of a deformed ridge shape are located on a common imaginary reference cylindrical surface (S) of cylindrical shape about a center axis (11), with an inner wall-side protruding shape of a cross section of each reference ring (20) taken along the center axis (11) being defined as the reference ridge shape, and with an inner wall-side protruding shape of a cross section of each deformed ring (300) taken along the center axis (11) being defined as the deformed ridge shape; and
the deformed ridge shape has a ridge height lower than that of the reference ridge shape.

2. The medical tube (1) accordinq to claim 1, wherein the inner diameters of the inner wall-side innermost parts of the tube end portions (31,31A,31B) are the same as a diameter of the imaginary reference cylindrical surface (S).

3. The medical tube according to claim 1 or 2, wherein the reference rings and the deformed rings have the same axial width.

4. The medical tube (1) according to any one of claims 1 to 3, wherein the deformed ridge shape has a large crest angle or curvature compared to that of the reference ridge shape.

5. The medical tube (1) according to any one of claims 1 to 4, wherein at least one of the tube end portions (31,31A,31B) between adjoining ones of the medical tube pieces (10) includes a connection portion (31C) that, if cut, takes a shape connectable to a medical member.

6. The medical tube (1) according to any one of claims 1 to 5, comprising at least three deformed side bellows tube portions (30), wherein
at least three deformed side bellows tube portions (30) arranged from one side to the other in the axial direction (L) with the reference side bellows tube portions (2) interposed therebetween increase or decrease in the axial length in order.

7. The medical tube (1) according to any one of claims 1 to 5, comprising at least three deformed side bellows tube portions (30), wherein
at least three deformed side bellows tube portions (30) arranged from one side to the other in the axial direction (L) with the reference side bellows tube portions (2) interposed therebetween increase or decrease in the number of deformed rings (300) in order.
